# FASCICULE DE BREVET EUROPEEN

(11) **EP 0 627 414 B1**
(45) Date de publication et mention de la délivrance du brevet: **16.04.1997**
(21) Numéro de dépôt: 94401112.1
(22) Date de dépôt: 19.05.1994
(51) Int. Cl.: C07C 303/16, C07C 309/04

(54) **Procédé de préparation d'acides alcanesulfoniques**
Verfahren zur Herstellung von Alkansulfonsäure
Method for the preparation of alkanesulphonic acids

(30) Priorité: 02.06.1993 FR 9306560
(43) Date de publication de la demande: 07.12.1994
(73) Titulaire: ELF ATOCHEM S.A., 92800 Puteaux (FR)
(72) Inventeur: Lacombe, Sylvie, F-64230 Artiguelouve (FR); Ollivier, Jean, F-64260 Arudy (FR)
(74) Mandataire: Leboulenger, Jean

(56) Documents cités:
- US-A- 2 697 722
- US-A- 3 392 095
- JOURNAL OF ORGANIC CHEMISTRY, vol. 37, no. 22, 3 novembre 1972, Washington, DC, US, pages 3516 - 3520, R.W. MURRAY et al: "Photosensitised oxidation of dialkyl disulphides"

## Description

La présente invention concerne la synthèse d'acides alcanesulfoniques et a plus particulièrement pour objet la préparation de ces acides à partir des dialkyl disulfures correspondants.

Les acides alcanesulfoniques et leurs sels ont de nombreuses applications industrielles, notamment comme détergents, émulsifiants, catalyseurs d'estérification, durcisseurs pour certaines résines.

Industriellement, les acides alcanesulfoniques sont le plus souvent fabriqués à partir des alcanes par sulfooxydation ou par sulfochloration. Ces deux voies de synthèse présentent cependant l'inconvénient de conduire à la formation de sous-produits sulfonés sur les différents atomes de carbone de la chaîne hydrocarbonée. D'autre part, l'hydrolyse des alcanesulfochlorures conduit généralement à des acides alcanesulfoniques plus ou moins colorés, nécessitant un traitement final de décoloration, par exemple au moyen de chlore.

Ces dernières années, de nombreux travaux ont été consacrés à la photochimie atmosphérique de composés organiques soufrés (thiols, sulfures, disulfures) qui sont, à l'état de traces, des constituants atmosphériques d'origine biogénique ou, plus souvent, des polluants issus des technologies de production d'énergie. La plupart de ces études photochimiques ont été effectuées en phase gazeuse sous faible pression (≤ 1 atm) en présence d'air, d'oxygène, d'un gaz éluant et parfois d'oxydes d'azote. Tous les résultats obtenus mentionnent la formation de dioxyde de soufre, d'acides sulfonique et sulfurique, ainsi que d'autres produits variant selon les conditions mises en oeuvre.

Les recherches sur la photooxydation en solution de composés organiques soufrés ont été presque uniquement consacrées à la photooxydation des sulfures en présence de photosensibilisants pour la préparation de sulfoxydes et de sulfones. Un seul article (R.W. MURRAY et al., J. Org. Chem., 37, 1972, p.3516) concerne la formation de thiolsulfinates par photooxydation par de l'oxygène des disulfures en solution dans le méthanol en présence de bleu de méthylène comme photosensibilisant.

Le brevet US 3 392 095 décrit un procédé pour transformer un disulfure d'alkyle R'-S-S-R'' en acide sulfonique R'-SO₃H ou R''-SO₃H par oxydation par l'oxygène en présence d'une quantité catalytique d'un oxyde d'azote choisi parmi NO, NO₂, N₂O₃, N₂O₄, N₂O₅, d'un solvant choisi parmi les hydrocarbures chlorés et les hydrocarbures aromatiques, et d'environ 0,001 à environ 25% en poids d'eau basé sur l'oxyde d'azote, la réaction étant initiée par de la lumière actinique. Il a maintenant été trouvé que d'excellents rendements en acides alcanesulfoniques peuvent être obtenus par photooxydation des dialkyl disulfures en présence d'oxygène sans catalyseur si l'on opère en solution dans un alcool avec une source lumineuse irradiant entre 200 et 320 nm. Ce procédé qui met en oeuvre la réaction suivante : 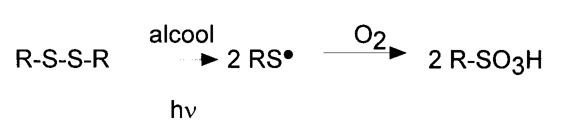 présente en outre l'avantage de conduire à des acides dont le groupement sulfonique est exclusivement fixé à l'extrémité de la chaîne hydrocarbonée.

L'invention a donc pour objet un procédé de préparation d'un acide alcanesulfonique R-SO₃H à partir du dialkyl disulfure R-S-S-R correspondant, caractérisé en ce qu'il consiste à soumettre une solution alcoolique du dialkyl disulfure ne comportant pas de bleu de méthylène comme photosensibilisant, en présence d'oxygène, à une irradiation par des rayonnements lumineux de longueur d'onde comprise entre 200 et 320 nm.

Le procédé selon l'invention peut s'appliquer à la synthèse des acides alcanesulfoniques dont la chaîne hydrocarbonée, linéaire ou ramifiée, peut contenir de 1 à 16 atomes de carbone, en particulier de 1 à 4 atomes de carbone. Comme exemples de dialkyl disulfures à utiliser comme produits de départ, on peut mentionner, à titre non limitatif, le diméthyl disulfure, le diéthyl disulfure, le dipropyl disulfure et le dibutyl disulfure.

L'alcool utilisé peut être avantageusement choisi parmi les alcools primaires, secondaires ou tertiaires contenant de 1 à 12 atomes de carbone. On préfère cependant utiliser un alcool en C₁ à C₄ et, plus particulièrement, le méthanol. La teneur en dialkyl disulfure de la solution alcoolique de départ peut varier dans de larges limites en fonction de l'alcool et du disulfure mis en oeuvre. Elle peut généralement aller de 0,1 à 90 % en poids, mais est de préférence comprise entre 2 et 25 %.

L'oxygène, nécessaire à la réaction, peut être fourni sous forme pure ou diluée par un gaz inerte tel que, par exemple, l'azote. L'oxygène est de préférence introduit progressivement au sein de la solution alcoolique. La quantité totale d'oxygène nécessaire est d'au moins 4 moles par mole de dialkyl disulfure présent dans la solution initiale, mais on préfère opérer avec un excès d'oxygène d'au moins 50 %.

La photooxydation selon l'invention peut être menée à une température comprise entre -20°C et le point d'ébullition de l'alcool, mais on opère de préférence entre 18 et 45°C. L'opération est avantageusement réalisée à la pression atmosphérique, mais on ne sortirait pas du cadre de la présente invention en travaillant sous légère pression.

La procédé selon l'invention peut être mis en oeuvre de manière discontinue ou continue, dans tout réacteur photochimique, par exemple dans un réacteur à immersion ou à film tombant, équipé d'une ou plusieurs lampes à vapeur de mercure à basse, moyenne ou haute pression, ou de lampes eximères émettant dans l'ultraviolet.

Les exemples suivants illustrent l'invention, sans la limiter.

### EXEMPLE 1

***a)*** On utilise un réacteur photochimique équipé d'un porte-lampe central à immersion. La source lumineuse est une lampe Hanovia, moyenne pression de mercure, 450 W (référence 679 A) et le porte-lampe utilisé est en quartz. Avec ce porte-lampe, le volume de liquide dans le réacteur est de 300 ml et le trajet optique de 10 mm. Le réacteur et le porte-lampe sont thermostatés par deux groupes froids indépendants (15°C pour le réacteur et 5°C pour le porte-lampe). L'oxygène est introduit à pression atmosphérique par le bas du réacteur à travers un fritté (porosité 4). Pour minimiser l'entraînement des produits volatiles, l'effluent gazeux en sortie du réacteur passe à travers un réfrigérant refroidi à -20°C ; il est ensuite admis dans un barboteur à soude et enfin dirigé vers une torche.
   Dans le réacteur, on introduit 300 ml d'une solution 0,56 M de diméthyldisulfure (DMDS) dans le méthanol (soit 0,168 mole de DMDS), puis on laisse la solution sous bullage d'oxygène (débit : 1 litre/heure) pendant 2 heures à 10°C. On allume ensuite la lampe et, tout en maintenant le bullage d'oxygène (2 l/h), on irradie pendant 14 heures à 35-40°C.
   Après cette période, la solution est claire et homogène et on ne remarque la présence d'aucun précipité. Après évaporation du solvant, on obtient un liquide incolore dont l'analyse par RMN¹H indique une conversion quasi-totale (99 %) du DMDS avec un rendement de 65 % en acide méthanesulfonique (AMS).
   Le principal sous-produit de la réaction est l'acide sulfurique (AS) (rendement 18 %) qui s'estérifie partiellement dans le méthanol en hydrogénométhylester (HMAS). Parmi les sous-produits détectés en faible quantité, on remarque la présence de l'ester méthylique de l'acide méthanesulfonique (MMS : 2 %), de diméthylsulfone (DMSO₂ : 0,6 %) et de traces d'ester méthylique de l'acide méthanesulfinique (MMSI), mais on note l'absence de méthanethiosulfonate de méthyle (MMTS).
   ***b)*** On opère comme précédemment, mais en remplaçant la solution méthanolique par du DMDS pur (300 ml). L'irradiation en présence d'oxygène conduit rapidement à l'obtention d'un liquide non homogène et opalescent, nécessitant l'arrêt de l'opération au bout de 2 heures. L'AMS n'est présent qu'à l'état de traces dans la phase lourde séparée par décantation. L'analyse des effluents volatils indique la formation de dioxyde de soufre, de méthane et de méthylmercaptan.

### EXEMPLES COMPARATIFS 2 à 4

On répète l'opération de l'exemple 1a, mais en remplaçant le méthanol par le cyclohexane (exemple 2) ou l'acétonitrile (exemple 3) ou en utilisant un porte-lampe en Pyrex (exemple 4). Avec ce porte-lampe, le volume de liquide dans le réacteur est de 200 ml et le trajet optique de 8 mm.

Le tableau suivant rassemble les résultats obtenus dans ces essais et rappelle pour comparaison ceux de l'exemple 1a.

| **EXEMPLE** | **1a** | **2** | **3** | **4** |
|---|---|---|---|---|
| | | | | |

| *Conditions opératoires* | | | | |
|---|---|---|---|---|
| Solvant | méthanol | cyclohexane | acétonitrile | méthanol |
| Concentration initiale en DMDS | 0,56 M | 0,56 M | 0,56 M | 0,49 M |
| Porte-lampe | quartz | quartz | quartz | Pyrex |
| Durée d'irradiation (h) | 14 | 11,5 | 5,25 | 9,5 |

| *Résultats* : | | | | |
|---|---|---|---|---|
| Taux de conversion du DMDS (%) | 99 | 50 | 40 | 21 |

| Rendement (%) en : | | | | |
|---|---|---|---|---|
| • AMS | 65 | 4 | 5,7 | 7,1 |
| • MMS | 2 | - | - | traces |
| • DMSO₂ | 0,6 | - | - | traces |
| • MMSI | traces | - | - | traces |
| • AS + HMAS | 18 | 0,3 | 0,3 | Non déterminé |
| • MMTS | 0 | 1,2 | 5,6 | 6,2 |

L'examen des résultats permet d'apprécier l'importance du solvant et du filtre utilisés sur le taux de conversion du DMDS, le rendement en AMS et la formation indésirable de MMTS.

## Revendications

1. Procédé de préparation d'un acide alcanesulfonique à partir du dialkyl disulfure correspondant, caractérisé en ce qu'il consiste à soumettre une solution alcoolique du dialkyl disulfure ne comportant pas de bleu de méthylène comme photosensibilisant, en présence d'oxygène, à une irradiation par des rayonnements lumineux de longueur d'onde comprise entre 200 et 320 nm.

2. Procédé selon la revendication 1, dans lequel les radicaux alkyle du dialkyl disulfure sont des radicaux alkyle, linéaires ou ramifiés, contenant de 1 à 16, de préférence 1 à 4 atomes de carbone.

3. Procédé selon la revendication 1 ou 2, dans lequel le solvant est un alcool en C₁ à C₄, de préférence le méthanol.

4. Procédé selon l'une des revendications 1 à 3, dans lequel la teneur en dialkyl disulfure de la solution alcoolique de départ est comprise entre 0,1 et 90 % en poids, de préférence entre 1 et 25 %.

5. Procédé selon l'une des revendications 1 à 4, dans lequel on opère à une température comprise entre -20°C et le point d'ébullition de l'alcool, de préférence entre 18 et 45°C.

6. Procédé selon l'une des revendications 1 à 5, dans lequel on opère à pression atmosphérique.

7. Application du procédé selon l'une des revendications 1 à 6 à la synthèse de l'acide méthanesulfonique à partir du diméthyldisulfure.

## Claims

1. Process for the preparation of an alkanesulphonic acid from the corresponding dialkyl disulphide, characterized in that it consists in submitting an alcoholic solution of the dialkyl disulphide containing no methylene blue as photosensitizer, in the presence of oxygen, to an irradiation by light rays of wavelength between 200 and 320 nm.

2. Process according to Claim 1, in which the alkyl radicals of the dialkyl disulphide are linear or branched alkyl radicals containing from 1 to 16, preferably from 1 to 4 carbon atoms.

3. Process according to Claim 1 or 2, in which the solvent is a C₁ to C₄ alcohol, preferably methanol.

4. Process according to one of Claims 1 to 3, in which the dialkyl disulphide content of the starting alcoholic solution is between 0.1 and 90 % by weight, preferably between 1 and 25 %.

5. Process according to one of Claims 1 to 4, in which the procedure is carried out at a temperature between -20°C and the boiling point of the alcohol, preferably between 18 and 45°C.

6. Process according to one of Claims 1 to 5, in which the procedure is carried out at atmospheric pressure.

7. Application of the process according to one of Claims 1 to 6 to the synthesis of methanesulphonic acid from dimethyl disulphide.

## Patentansprüche

1. Verfahren zur Herstellung von Alkansulfonsäuren aus dem entsprechenden Dialkyldisulfid,
dadurch gekennzeichnet, daß
es darin besteht, eine alkoholische Lösung von Dialkyldisulfid, die kein Methylenblau als Photosensibilisator enthält, in Gegenwart von Sauerstoff einer Bestrahlung mit Lichtstrahlen einer Wellenlänge im Bereich von 200 bis 320 nm auszusetzen.

2. Verfahren nach Anspruch 1, worin die Alkylgruppen des Dialkyldisulfids geradkettige oder verzweigte Alkylgruppen mit 1 bis 16 Kohlenstoffatomen und vorzugsweise 1 bis 4 Kohlenstoffatomen sind.

3. Verfahren nach Anspruch 1 oder 2, worin das Lösungsmittel ein Alkohol mit 1 bis 4 Kohlenstoffatomen und vorzugsweise Methanol ist.

4. Verfahren nach einem der Ansprüche 1 bis 3, worin der Gehalt an Dialkyldisulfid der alkoholischen Ausgangslösung im Bereich von 0,1 bis 90 Gew.-% und vorzugsweise von 1 bis 25 Gew.-% liegt.

5. Verfahren nach einem der Ansprüche 1 bis 4, worin bei einer Temperatur im Bereich von -20 °C bis zum Siedepunkt des Alkohols und vorzugsweise von 18 bis 45 °C gearbeitet wird.

6. Verfahren nach einem der Ansprüche 1 bis 5, worin unter Atmosphärendruck gearbeitet wird.

7. Verwendung des Verfahrens nach einem der Ansprüche 1 bis 6 zur Synthese von Methansulfonsäure aus Dimethyldisulfid.
